# EUROPÄISCHE PATENTSCHRIFT

(11) **EP 1 254 122 B1**
(45) Veröffentlichungstag und Bekanntmachung des Hinweises auf die Patenterteilung: **17.09.2008**
(21) Anmeldenummer: 00985213.8
(22) Anmeldetag: 20.12.2000
(51) Int. Cl.: C07D 253/08, A61K 31/53, A61P 35/00

(54) **NEUE 3-SUBSTITUIERTE 1,2,4-BENZOTRIAZINE, VERFAHREN ZU IHRER HERSTELLUNG UND IHRE VERWENDUNG ZUR BEHANDLUNG UND PROPHYLAXE VON TUMORERKRANKUNGEN**
NOVEL 3-SUBSTITUTED 1,2,4-BENZOTRIAZINES, A METHOD FOR THEIR PRODUCTION AND THE USE THEREOF
NOUVELLE 1,2,4-BENZOTRIAZINE SUBSTITUEE EN 3, SON PROCEDE DE PRODUCTION ET SON UTILISATION

(30) Priorität: 21.12.1999 DE 19963316; 30.06.2000 US 215756 P
(43) Veröffentlichungstag der Anmeldung: 06.11.2002
(73) Patentinhaber: UFZ-UMWELTFORSCHUNGSZENTRUM Leipzig-Halle GmbH, 04318 Leipzig (DE)
(72) Erfinder: STOTTMEISTER, Ulrich, 04349 Leipzig (DE); SCHÖNFELDER, Manfred, 04229 Leipzig (DE); WILDE, Horst, 04277 Leipzig (DE); SICKER, Dieter, 04107 Leipzig (DE); ANDERSCH, Jens, 09648 Mittweida (DE)
(74) Vertreter: Ziebig, Marlene
(86) Internationale Anmeldenummer: PCT/EP2000/013028
(87) Internationale Veröffentlichungsnummer: WO 2001/046162

(56) Entgegenhaltungen:
- EP-A- 0 649 658
- DD-A- 150 463
- DD-A- 278 791
- REICH M F ET AL: "Pyrido[3,4-e]-1,2,4-triazines and Related Heterocycles as Pontential Antifungal Agents" JOURNAL OF MEDICINAL CHEMISTRY., Bd. 32, Nr. 11, 1989, Seiten 2474-2485, XP002167214 ISSN: 0022-2623
- DATABASE XFIRE [Online] Beilstein Informationsysteme GmbH, Frankfurt, DE; die Verbindung mit der BRN: 126057, XP002167215 & REND. IST. LOMB., Bd. 91, 1957, Seiten 936-944,
- DATABASE XFIRE [Online] Beilstein Informationssysteme GmbH, Frankfurt, DE; die Verbindung mit der BRN: 126400, XP002167216 & CHEM. BER., Bd. 22, 1889, Seite 2818
- DATABASE XFIRE [Online] Beilstein Informationssysteme GmbH, Frankfurt, DE; die Verbindung mit der BRN: 125771, XP002167217 & J. CHEM. SOC., 1955, Seiten 2326-2334,
- HENRIE R N, II ET AL: "Discovery and optimization of a PSI electron-accepting 1,2,4-benzotriazine herbicide" QUANT. STRUCT.-ACT. RELAT., Bd. 12, Nr. 1, 1993, Seiten 27-37, XP000996799
- DATABASE XFIRE [Online] Beilstein Informationssysteme GmbH, Frankfurt, DE; die Verbindung mit der BRN: 5961539, XP002167218 & J. PRAKT. CHEM., Bd. 327, Nr. 2, 1985, Seiten 297-309,
- ANDERSCH J ET AL: "Reductive cyclization of carbohydrate 2-nitrophenylhydrazones to chiral functionalized 1,2,4-benzotriazines and benzimidazoles" J. HETEROCYCL. CHEM., Bd. 36, Nr. 3, 1999, Seiten 589-594, XP000999073

## Beschreibung

Die Erfindung betrifft die Verwendung von 3-substituierten 1,2,4-Benzotriazinen der allgemeinen Formel I zur Herstellung von Arzneimitteln zur Behandlung und Prophylaxe von Tumorerkrankungen. Gegenstand der Erfindung sind auch neue 1,2,4-Benzotriazine sowie Verfahren zur Herstellung dieser 3-substituierten 1,2,4-Benzotriazine.

Verbindungen, die eine anticancerogene Wirksamkeit aufweisen, sind zahlreich bekannt. In der Klasse der 1,2,4-Triazine sind cancerostatische Wirksamkeiten von 3-Amino- und 3-Alkyl-1,2,4-benzotriazin-1,4-dioxiden beschrieben. So haben z.B. Monge, Antonio et al. in J. Med. Chem.; EN, 38, 10, 1995; 1786-1792 und in J. Med. Chem.; EN; 38, 22, 1995; 4488-4494 cytotoxische Untersuchungen mit 3-Amino-1,2,4-benzotriazin-1,4-dioxid an V-79 Zellen (Chinesische Hamster-Lungenfibroblasten) dargestellt. Die Synthese und antitumorale Aktivität weiterer neuer Analoga wurde von Tracy, M. auf einem Workshop der British Association of Cancer in Oxford, England (17.-19. September 1992) gezeigt. Die antitumorale Wirksamkeit von 3-Alkyl-1,2,4-benzotriazin-1,4-dioxiden war Gegenstand auf dem VIII. Meeting über chemische Modifikatoren zur Krebsbehandlung in Kyoto, Japan vom 16-19.06.1993. Desweiteren ist auch eine cancerostatische Wirksamkeit von 4-Amino-3-hydrazino-6-methyl-4(H)-1,2,4-triazin-5-on bekannt (Borrell, J.I. et al. ANQUEX; An. Quim.; EN; 91; 3-4: 1995; 243-252).
Aus EP-A 0649658 sind 1,2,4-Benzotriazin-1-oxide bekannt, welchen eine cytotoxische Wirkung im Hinblick auf hypoxische Tumorzellen zugeschrieben wird.

Der Erfindung lag die Aufgabe zugrunde, weitere cytostatisch wirksame Verbindungen zu finden, die in die Zelle eingeschleust werden können sowie die Proliferation von Tumorzellen hemmen und damit zur Herstellung von Arzneimitteln zur Behandlung und Prophylaxe von Tumorerkrankungen geeignet sind. Aufgabe der Erfindung war es außerdem, ein Verfahren zu deren Herstellung zu entwickeln, das hinsichtlich Reaktionsführung einfach ist und die Verbindungen in guten Ausbeuten zur Verfügung stellt.

Die Aufgabe wird gemäß den Ansprüchen gelöst. Gegenstand der Erfindung ist die Verwendung von 3-substituierten 1,2,4-Benzotriazinen der allgemeinen Formel I 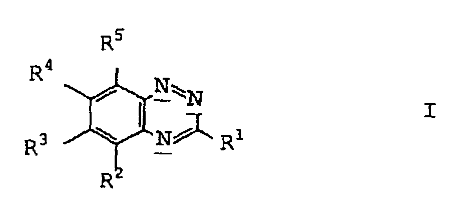 worin
- R¹: einen Alkyl- oder Alkyl-COO-Alkyl-Rest,
einen mit Hydroxygruppen substituierten bzw. einen mit Hydroxygruppen und -COO-Alkylgruppen substituierten Alkyl-Rest (Polyolyl-Rest), einen Alkyl-CO-NH-Alkylrest oder einen durch Morpholin-4-yl-CO-substituierten Polyolylrest bedeutet, wobei Alkyl jeweils für einen Rest mit einer Kettenlänge von 1 bis 6 C-Atomen steht,
und
- R², R³, R⁴, R⁵: gleich oder verschieden voneinander Wasserstoff, Halogen, NH₂ bedeuten.

Die Verbindungen der allgemeinen Formel I sind biologisch aktiv und besitzen eine ausgeprägte Antitumorwirksamkeit. Insbesondere lassen sie sich somit vorteilhaft zur Herstellung von Arzneimitteln zur Behandlung und Prophylaxe von Tumorerkrankungen einsetzen.

Insbesondere bevorzugt verwendete Verbindungen sind solche, in denen
- R¹: -CH₃, -CH₂-COO-CH₃, -CH₂-COO-C₂H₅,
D-ribo-(CHOH)₃-CH₂OH,
D-galacto-(CHOH)₄-CH₂OH, D-galacto-(CHOH)₄-COO-C₂H₅,
D-arabino-(CHOH)₃-CH₂OH,
-CH₂-CO-NH-CH(CH₃)₂, L-arabino-(CHOH)₄-CO-N O
bedeutet und
- R², R³, R⁴, R⁵: gleich oder verschieden voneinander H, Cl, Br und NH₂ bedeuten.

Ganz bevorzugt verwendete Verbindungen sind:
a) 3-Methyl-1,2,4-benzotriazin (R¹ = -CH₃, R², R³, R⁴, R⁵ = H),
b) 3-(Methoxy-carbonylmethyl)-6-chlor-benzo-1,2,4-triazin (R¹ = -CH₂-CO-OCH₃, R², R³, R⁵ = H und R⁴ = Cl),
c) 3-(Methoxy-carbonylmethyl)-5-chlor-benzo-1,2,4-triazin (R¹ = -CH₂-CO-OCH₃, R², R⁴, R⁵ = H und R³ = Cl),
d) 3-(Methoxy-carbonylmethyl)-5-amino-6-chlor-benzo-1,2,4-triazin (R¹ = -CH₂-CO-OCH₃, R³ = NH₂, R⁴ = Cl, R², R⁵ = H),
e) 3-(Methoxy-carbonylmethyl)-1,2,4-benzotriazin (R¹ = -CH₂-CO-OCH₃ und R², R³, R⁴, R⁵ = H),
f) 3-(Ethoxy-carbonylmethyl)-1,2,4-benzotriazin (R¹ = -CH₂-CO-OC₂H₅ und R², R³, R⁴, R⁵ = H),
g) 3-(Ethoxy-carbonylmethyl)-6-brom-benzo-1,2,4-triazin (R¹ = -CH₂-CO-OC₂H₅, R², R³, R⁵ = H, R⁴ = Br),
h) 3-(D-arabino-Tetritol-1-yl)-1,2,4-benzotriazin (R¹ = D-arabino-(CHOH)₃-CH₂OH, R², R³, R⁴, R⁵ = H),
i) 3-(D-galacto-Tetritol-1-yl)-1,2,4-benzotriazin (R¹ = D-galacto-(CHOH)₄-CH₂OH, R², R³, R⁴, R⁵ = H),
j) 3-(Ethoxycarbonyl-D-galacto-tetritol-1-yl-)-1,2,4-benzotriazin
   (R¹ = D-galacto-(CHOH)₄-CO-OC₂H₅, R², R³, R⁴, R⁵ = H),
k) 3-(D-arabino-Tetritol-1-yl)-6-chlor-benzo-1,2,4-triazin (R¹ = D-arabino- (CHOH)₃-CH₂OH, R², R³, R⁵ = H, R⁴ = Cl),
l) 3-(Isopropylamino-carbonylmethyl)-1,2,4-benzotriazin (R¹ = -CH₂-CO-NH-CH(CH₃)₂, R², R³, R⁴, R⁵ = H),
m) 3-(D-ribo-Tetritol-1-yl)-1,2,4-benzotriazin (R¹ = D-ribo-(CHOH)₃-CH₂OH, R², R³, R⁴, R⁵ = H),
n) 3-(Morpholin-4-yl-carbonyl-L-arabino-tetritol-1-yl)-1,2,4-benzotriazin
   (R¹ = L-arabino-(CHOH)₄-CO-N O, R², R³, R⁴, R⁵ = H).

Gegenstand der Erfindung sind weiterhin 1,2,4-Benzotriazine der allgemeinen Formel I, 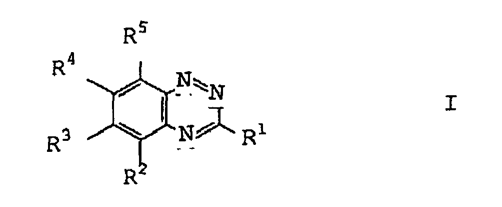 worin
- R¹: D-ribo-(CHOH)₃-CH₂OH,
-CH₂-CO-NH-CH(CH₃)₂
oder 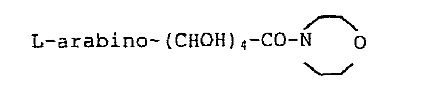 ist und
- R², R³, R⁴, R⁵: gleich oder verschieden voneinander H, Cl, Br, NH₂ bedeuten.

Weiterhin Gegenstand der Erfindung sind die 1,2,4-Benzotriazine:
3-(Methoxy-carbonylmethyl)-5-chlor-benzo-1,2,4-triazin
3-(Methoxy-carbonylmethyl)-5-amino-6-chlor-benzo-1,2,4-triazin
3-(D-arabino-Tetritol-1-yl)-6-chlor-benzo-1,2,4-triazin
3-(Isopropylamino-carbonylmethyl)-1,2,4-benzotriazin
3-(D-ribo-Tetritol-1-yl)-1,2,4-benzotriazin
3-(Morpholin-4-yl-carbonyl-L-arabino-tetritol-1-yl)-1,2,4-benzotriazin.

Die Verbindungen der allgemeinen Formel I können nach an sich bekannten Verfahren synthetisiert werden (z.B. Jerschel, D. und W. Edler in Chem. Ber. 88 (1955) 1284; Fusco, R. und G. Bianchetti, Gazz. Chim. Ital. 90 (1960) 1113 oder Kwee, S. und H. Lund, Acta Chem. Scand. 23 (1969) 2711), indem 2-Nitrophenylhydrazone einer Carbonylverbindung oder die tautomeren Azoverbindungen reduziert werden.

Erfindungsgemäß werden sie durch Umsetzung eines entsprechenden 2-Nitrophenylhydrazins mit einer entsprechenden 2-Oxocarbonsäure oder dessen Salz gemäß dem folgenden Formelschema unter Entstehen des 2-Nitrophenylhydrazons erhalten. Das entstandene 2-Nitrophenylhydrazon wird anschließend mittels Wasserstoff katalytisch zu einer Zielverbindung der allgemeinen Formel I reduziert. 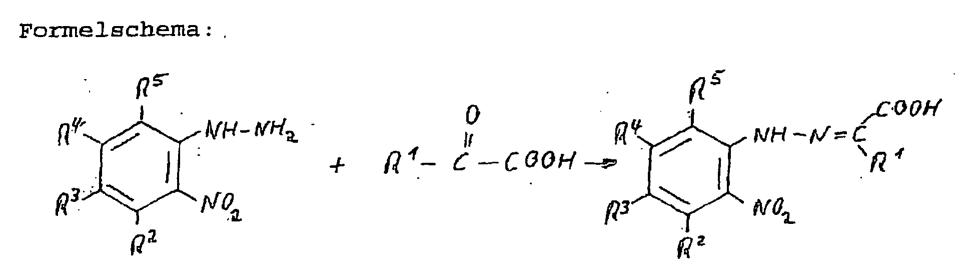 wobei R¹ bis R⁵ die o.g. Bedeutung besitzen.

Die eingesetzten substituierten oder unsubstituierten 2-Nitrophenylhydrazine sind an sich bekannt und werden durch Reduktion von Diazoniumverbindungen hergestellt (Mangini, Deliddo in Gazz. Chim. Ital. 63, 612, 1933) oder durch Umsetzung von 2-Nitro-halogenbenzenen mit Hydrazinhydrat (Albini, Angelo et al. in Heterocycles 597, 1995).

In einer bevorzugten Ausführungsvariante des Verfahrens werden für die Synthese der 2-Nitrophenylhydrazone direkt Fermentationslösungen eingesetzt, die eine entsprechende 2-Oxocarbonsäure enthalten. Das gilt bevorzugt für Fermentationslösungen, die 2-Oxoglutarsäure oder 2-Oxogluconsäure enthalten.

Der für eine Umsetzung notwendige Gehalt einer 2-Oxocarbonsäure in der Fermentationslösung beträgt in der Regel ca. 15%, vorzugsweise >20%.

Das umzusetzende 2-Nitrophenylhydrazin wird dabei in einem organischen Lösungsmittel bzw. -gemisch, ggf. unter Erwärmen, gelöst und einer die entsprechende 2-Oxocarbonsäure enthaltenden Fermentationslösung bei einer Temperatur von 20 bis 80°C, vorzugsweise bei 45-55°C zugegeben. Das entstandene 2-Nitrophenylhydrazon wird ggf. aufgearbeitet. Durch katalytische Reduktion werden anschließend unter Cyclisierung, Decarboxylierung und Oxidation die Zielverbindungen der allgemeinen Formel I mit hohen Ausbeuten > 30%, vorzugsweise > 50 % erhalten.

Dieses Verfahren hat den großen Vorteil, dass es in einer Eintopfreaktion ablaufen kann und somit hinsichtlich Reaktionsführung und auch hinsichtlich der erzielten Ausbeuten den bekannten Synthesewegen, deren Ausbeuten in der Regel unter 30 % liegen, überlegen ist.

Anschließend wird die Erfindung an Ausführungsbeispielen näher erläutert.

### Beispiel 1

### 3-Methyl-1,2,4-benzotriazine

0,01 mol Natriumpyruvat und 0,01 mol des entsprechenden 2-Nitrophenylhydrazins werden in einer Lösung von 10 ml Ethanol und 10 ml 35 proz. Perchlorsäure unter Rühren zur Reaktion gebracht. Der nach kurzer Zeit anfallende gelbe Niederschlag wird abgesaugt, mit Wasser neutral gewaschen, getrocknet und anschließend aus Ethanol umkristallisiert.
Ausbeuten: 90-95% der Theorie

3 mmol der so erhaltenen 2-Nitrophenylhydrazone werden in 50 ml Ethanol gelöst und über Platinoxid bei Raumtemperatur und Normaldruck bis zum Ende der Wasserstoffaufnahme hydriert. Danach wird der Katalysator abfiltriert und das Lösungsmittel im Vakuum abdestilleirt. Die verbleibenden 3-Methyl-1,2,4-benzotriazine werden aus Petrolether umkristallisiert.
Ausbeuten: 55-70% der Theorie.

### Beispiel 2

### 3-(D-arabino-Tetritol-1-yl)-1,2,4-benzotriazin

10 mmol 2-Nitrophenylhydrazin werden in 30 ml Methanol und 0,5 ml Essigsäure unter Erwärmen gelöst. Die Lösung wird bei 50°C unter Rühren zu einer Fermentationslösung getropft, die einen Gehalt von 24% 2-Oxo-D-gluconsäure aufweist. Die Reaktionsmischung wird eine weitere Stunde gerührt. Der orange Niederschlag wird abgesaugt, gewaschen und aus Wasser umkristallisiert.
Ausbeuten: 90% der Theorie.

Eine Suspension von 6 mmol des so erhaltenen 2-Nitrophenylhydrazons in 60 ml Tetrahydrofuran wird über Pt/C unter Normaldruck bei Raumtemperatur bis zur berechneten Wasserstoffaufnahme hydriert. Das Reaktonsgemisch wird 3 Tage an der Luft gerührt. Danach wird das Lösungsmittel abgetrennt und das Produkt durch Extraktion mit Methanol vom Katalysator getrennt.
Ausbeuten: 30% der Theorie; gelbe Kristalle F 173-175°C.

### Beispiel 3

### Hemmung des Zellwachstums durch die erfindungsgemäßen Verbindungen

Zu je 1 ml einer entsprechenden Zellstammlösung werden die zu testenden Substanzen in wäßriger steriler Lösung in Konzentrationsschritten von je 2-50 µg gespritzt. Die Testkammern werden anschließend zusammen mit einer unbehandelten Referenzkammer bei 37°C drei Tage entwickelt.

Die Auswertung erfolgt mittels Mikroskop. Es werden Zellvermehrung bzw. Zellzerstörung (Zustand der Zellmembran sowie des Zellkern) mit unbehandelten Referenzzellen verglichen.

**Tabelle: IC₅₀-Wert (50%ige Hemmung des Zellwachstums in µg/ml) ausgewählter Substanzen der Formel I**

| **Substanz** | **Hep2** | **HeLa** | **Peritoneal carcinom** | **Pleurocarcinom** |
|---|---|---|---|---|
| Verbindung b | 120 | 160 | 150 | 500 |
| Verbindung c | 16 | 8-16 | 12 | |
| Verbindung d | 500 | 50 | 25-30 | 500 |
| Verbindung e | 100 | 180 | 350 | 500 |
| Verbindung f | 190-200 | 378 | 180 | 500 |
| Verbindung g | 8-16 | 8-16 | 35 | |
| Verbindung h | 150 | 150 | | |
| Verbindung i | | 400 | | |
| Verbindung j | | | | |
| Verbindung k | | 250 | | |
| Verbindung l | 100 | 86 | 170 (100%) | |

Die erfindungsgemäßen Verbindungen hemmen sämtlich das Zellwachstum von permanenten Zellkulturen wie z.B. von HeLa-, Hep2-Zellen, von Peritonealcarcinom- sowie von Pleurocarcinomzellen.

Wie der obigen Tabelle zu entnehmen ist, sind alle Vebindungen wirksam, bevorzugte Wirksamkeit besitzen die Verbindungen c, g, l.

Es zeigte sich eine selektive Antitumorwirkung in vitro, d.h. das Wachstum von Normalzellen wird im vergleichbaren Konzentrationsbereich weit weniger gehemmt als das Tumorwachstum.

## Patentansprüche

1. Verwendung von 3-substituierten 1,2,4-Benzotriazinen der allgemeinen Formel I, 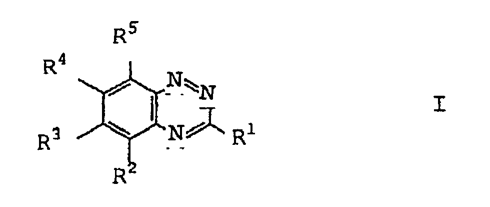 worin
R¹ einen Alkyl- oder Alkyl-COO-Alkyl-Rest, einen mit Hydroxygruppen substituierten bzw. einen mit Hydroxygruppen und -COO-Alkylgruppen substituierten Alkyl-Rest (Polyolyle), einen Alkyl-CO-NH-Alkylrest oder einen durch Morpholin-4yl-CO-substituierten Polyolylrest bedeutet, wobei Alkyl einen Rest mit jeweils 1 bis 6 C-Atomen darstellt und
R², R³, R⁴, R⁵ gleich oder verschieden voneinander Wasserstoff, Halogen, NH₂ bedeuten,
zur Herstellung von Arzneimitteln zur Behandlung und Prophylaxe von Tumorerkrankungen.

2. Verwendung nach Anspruch 1, **dadurch gekennzeichnet, dass** Verbindungen verwendet werden, in denen
R¹ -CH₃, -CH₂-COO-CH₃, -CH₂-COO-C₂H₅,
D-ribo-(CHOH)₃-CH₂OH,
D-galacto-(CHOH)₄-CH₂OH, D-galacto-(CHOH)₄-COO-C₂H₅,
D-arabino-(CHOH)₃-CH₂OH, -CH₂-CO-NH-CH(CH₃)₂ oder 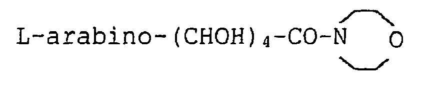 ist und
R², R³, R⁴, R⁵ gleich oder verschieden voneinander H, Cl, Br, NH₂ bedeuten.

3. Verwendung nach Anspruch 1 oder 2, **gekennzeichnet durch** Verwendung der folgenden Verbindungen:
a) 3-Methyl-1,2,4-benzotriazin
b) 3-(Methoxy-carbonylmethyl)-6-chlor-benzo-1,2,4-triazin
c) 3-(Methoxy-carbonylmethyl)-5-chlor-benzo-1,2,4-triazin
d) 3-(Methoxy-carbonylmethyl)-5-amino-6-chlor-benzo-1,2,4-triazin
e) 3-(Methoxy-carbonylmethyl)-1,2,4-benzotriazin
f) 3-(Ethoxy-carbonylmethyl)-1,2,4-benzotriazin
g) 3-(Ethoxy-carbonylmethyl)-6-brom-benzo-1,2,4-triazin
h) 3-(D-arabino-Tetritol-1-yl)-1,2,4-benzotriazin
i) 3-(D-galacto-Tetritol-1-yl)-1,2,4-benzotriazin
j) 3-(Ethoxycarbonyl-D-galacto-tetritol-1-yl-)-1,2,4-benzotriazin
k) 3-(D-arabino-Tetritol-1-yl)-6-chlor-benzo-1,2,4-triazin
l) 3-(Isopropylamino-carbonylmethyl)-1,2,4-benzotriazin
m) 3-(D-ribo-Tetritol-1-yl)-1,2,4-benzotriazin
n) 3-(Morpholin-4-yl-carbonyl-L-arabino-tetritol-1-yl)-1,2,4-benzotriazin.

4. 1,2,4-Benzotriazine allgemeinen Formel I, 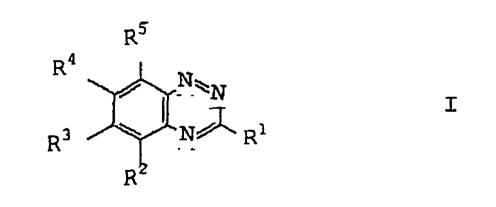 worin
R¹ D-ribo-(CHOH)₃-CH₂OH,
-CH₂-CO-NH-CH(CH₃)₂
oder 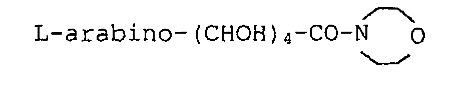 ist und
R², R³, R⁴, R⁵ gleich oder verschieden voneinander H, Cl, Br, NH₂ bedeuten.

5. 1,2,4-Benzotriazine, nämlich:
3-(Methoxy-carbonylmethyl)-5-chlor-benzo-1,2,4-triazin
3-(Methoxy-carbonylmethyl)-5-amino-6-chlor-benzo-1,2,4-triazin
3-(D-arabino-Tetritol-1-yl)-6-chlor-benzo-1,2,4-triazin
3-(Isopropylamino-carbonylmethyl)-1,2,4-benzotriazin
3-(D-ribo-Tetritol-1-yl)-1,2,4-benzotriazin
3-(Morpholin-4-yl-carbonyl-L-arabino-tetritol-1-yl)-1,2,4-benzotriazin.

6. Verfahren zur Herstellung eines 3-substituierten 1,2,4-Benzotriazins der allgemeinen Formel I gemäß Anspruch 4 oder 5,
**dadurch gekennzeichnet, dass** man
ein entsprechendes 2-Nitrophenylhydrazin mit einer entsprechenden 2-Oxocarbonsäure zum 2-Nitrophenylhydrazon umsetzt und dass man das erhaltene 2-Nitrophenylhydrazon anschließend mittels Wasserstoff katalytisch zu der Zielverbindung der allgemeinen Formel I reduziert.

7. Verfahren nach Anspruch 6, **dadurch gekennzeichnet, dass** das 2-Nitrophenylhydrazin mit einer Fermentationslösung, die eine 2-Oxocarbonsäure aufweist, zur Reaktion gebracht wird.

8. Verfahren nach Anspruch 7, **dadurch gekennzeichnet, dass** die Fermentationslösung die 2-Oxocarbonsäure mit einem Gehalt von mindestens 10%, vorzugsweise > 20% enthält.

## Claims

1. Use of 3-substituted 1,2,4-benzotriazines of general formula I, 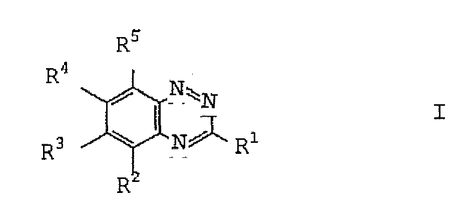 wherein
R¹ represents an alkyl or alkyl-COO-alkyl residue, an alkyl residue (polyolyl residue) substituted with hydroxy groups or substituted with hydroxy groups and -COO-alkyl groups, an alkyl-CO-NH-alkyl residue, or a polyolyl residue substituted with morpholin-4-yl-CO-, wherein each alkyl represents a residue having a chain length of from 1 to 6 C atoms,
R², R³, R⁴, and R⁵, same or different represent hydrogen, halogen, NH₂ in the production of drugs for the treatment and prophylaxis of tumor-related diseases.

2. Use according to claim 1, **characterized in that** compounds are used wherein
R¹ represents -CH₃, -CH₂COO-CH₃, -CH₂-COO-C₂H₅,
D-ribo-(CHOH)₃-CH₂OH,
D-galacto-(CHOH)₄-CH₂OH, D-galacto-(CHOH)₄-COO-C₂H₅,
D-arabino-(CHOH)₃-CH₂OH, -CH₂-CO-NH-CH(CH₃)₂ or 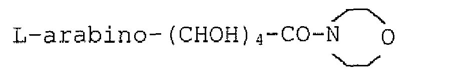 and
R², R³, R⁴, R⁵ same or different represent H, Cl, Br, NH₂.

3. Use according to claim 1 or 2, **characterized in that** compounds are used being:
a) 3-methyl-1,2,4-benzotriazine
b) 3-(methoxyocarbonylmethyl)-6-chloroenzo-1,2,4-triazine
c) 3-(methoxycarbonylmethyl)-5-chlorobenzo-1,2,4-triazine
d) 3-(methoxycarbonylmethyl)-5-amino-6-chlorobenzo-1,2,4-triazine
e) 3-(methoxycarbonylmethyl)-1,2,4-benzotriazine
f) 3-(ethoxycarbonylmethyl)-1,2,4-benzotriazine
g) 3-(ethoxycarbonylmethyl)-6-bromobenzo-1,2,4-triazine
h) 3-(D-arabino-tetritol-1-yl)-1,2,4-benzotriazine
i) 3-(D-galacto-tetritol-1-yl)-1,2,4-benzotriazine
j) 3-(ethoxycarbonyl-D-galacto-tetritol-1-yl-)-1,2,4-benzotriazine
k) 3-(D-arabino-tetritol-1-yl)-6-chlorobenzo-1,2,4-triazine
l) 3-(isopropylaminocarbonylmethyl)-1,2,4-benzotrzazine
m) 3-(D-ribo-tetritol-1-yl)-1,2,4-benzotriazine
n) 3-(morpholin-4-yl-carbonyl-L-arabino-tetritol-1-yl)-1,2,4-benzotriazine.

4. 1,2,4-benzotriazines of general formula I, 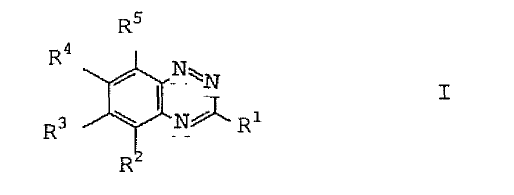 wherein
R¹ represents D-ribo-(CHOH)₃-CH₂OH,
-CH₂-CO-NH-CH(CH₃)₂
or 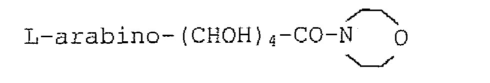 and
R², R³, R⁴, R⁵ same or different represent H, Cl, Br, NH₂.

5. 1,2,4-benzotriazines, namely:
3-(methoxymcarbonylmethyl)-5-chlorobenzo-1,2,4-triazine
3-(methoxycarbonylmethyl)-5-amino-6-chlorobenzo-1,2,4-triazine
3-(D-arabino-tetritol-1-yl)-6-chlorobenzo-1,2,4-triazine
3-(isopropylaminocarbonylmethyl)-1,2,4-benzotriazine
3-(D-ribo-tetritol-1-yl)-1,2,4-benzotriazine
3-(morpholin-4-yl-carbonyl-L-arabino-tetritol-1-yl)-1,2,4-benzotriazine.

6. A method of producing a 3-substituted 1,2,4-benzotriazine of general formula I according to any of claims 4 or 5,
**characterized in that**
an appropriate 2-nitrophenylhydrazine is reacted with an appropriate 2-oxocarboxylic acid to form the 2-nitrophenylhydrazone, and that the 2-nitrophenylhydrazone obtained is subsequently reduced catalytically using hydrogen to yield the target compound of general formula I.

7. The method according to claim 6,
**characterized in that**
the 2-nitrophenylhydrazine is reacted with a fermentation solution including a 2-oxocarboxylic acid.

8. The method according to claim 7,
**characterized in that**
the fermentation solution includes the 2-oxocarboxylic acid with a content of at least 10%, preferably >20%.

## Revendications

1. Utilisation de 1,2,4-benzotriazines 3-substituées de formule générale I, 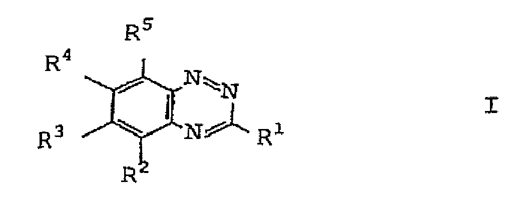 dans laquelle
R¹ représente un groupe alkyle ou alkyl-COO-alkyle, un groupe alkyle substitué avec des groupes hydroxy ou substitué avec des groupes hydroxy et des groupes -COO-alkyle (polyolyles), un groupe alkyl-CO-NH-alkyle ou un groupe polyolyle substitué par morpholin-4-yl-CO, moyennant quoi l'alkyle représente un groupe comportant à chaque fois de 1 à 6 atomes de carbone et
R², R³, R⁴, R⁵ représentent en étant identiques ou différents l'un de l'autre, de l'hydrogène, un halogène, NH₂,
pour préparer des médicaments destinés au traitement et à la prophylaxie des maladies tumorales.

2. Utilisation selon la revendication 1, **caractérisée en ce qu'**on utilise des composés, dans lesquels
R¹ est -CH₃, -CH₂-COO-CH₃, -CH₂-COO-C₂H₅,
D-ribo-(CHOH)₃-CH₂OH,
D-galacto-(CHOH)₄-CH₂OH, D-galacto-(CHOH)₄-COO-C₂H₅,
D-arabino-(CHOH)₃-CH₂OH, -CH₂-CO-NH-CH(CH₃)₂ ou 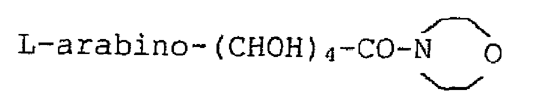 et
R², R³, R⁴, R⁵ représentent en étant identiques ou différents l'un de l'autre H, Cl, Br, NH₂.

3. Utilisation selon la revendication 1 ou 2, **caractérisée par** l'utilisation des composés suivants :
a) 3-méthyl-1,2,4-benzotriazine,
b) 3-(méthoxy-carbonylméthyl)-6-chloro-benzo-1,2,4-triazine,
c) 3-(méthoxy-carbonylméthyl)-5-chloro-benzo-1,2,4-triazine,
d) 3-(méthoxy-carbonylméthyl)-5-amino-6-chloro-benzo-1,2,4-triazine,
e) 3-(méthoxy-carbonylméthyl)-1,2,4-benzotriazine,
f) 3-(éthoxy-carbonylméthyl)-1,2,4-benzotriazine,
g) 3-(éthoxy-carbonylméthyl)-6-bromo-benzo-1,2,4-triazine,
h) 3-(D-arabino-tétritol-1-yl)-1,2,4-benzotriazine,
i) 3-(D-galacto-tétritol-1-yl)-1,2,4-benzotriazine,
j) 3-(éthoxycarbonyl-D-galacto-tétritol-1-yl)-1,2,4-benzotriazine,
k) 3-(D-arabino-tétritol-1-yl)-6-chloro-benzo-1,2,4-triazine,
l) 3-(isopropylamino-carbonylméthyl)-1,2,4-benzotriazine,
m) 3-(D-ribo-tétritol-1-yl)-1,2,4-benzotriazine,
n) 3-(morpholin-4-yl-carbonyl-L-arabino-tétritol-1-yl)-1,2,4-benzotriazine.

4. 1,2,4-Benzotriazines de formule générale I, 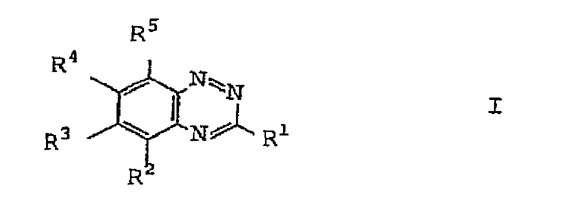 dans laquelle
R¹ est D-ribo-(CHOH)₃-CH₂OH,
-CH₂-CO-NH-CH(CH₃)₂
ou 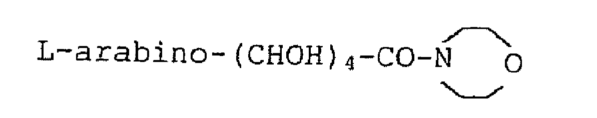 et
R², R³, R⁴, R⁵ représentent en étant identiques ou différents l'un de l'autre H, Cl, Br, NH₂.

5. 1,2,4-Benzotriazines, précisément :
3-(méthoxy-carbonylméthyl)-5-chloro-benzo-1,2,4-triazine,
3-(méthoxy-carbonylméthyl)-5-amino-6-chloro-benzo-1,2,4-triazine,
3-(D-arabino-tétritol-1-yl)-6-chloro-benzo-1,2,4-triazine,
3-(isopropylamino-carbonylméthyl)-1,2,4-benzotriazine,
3-(D-ribo-tétritol-1-yl)-1,2,4-benzotriazine,
3-(morpholin-4-yl-carbonyl-L-arabino-tétritol-1-yl)-1,2,4-benzotriazine.

6. Procédé de préparation d'une 1,2,4-benzotriazine 3-substituée de formule générale I selon la revendication 4 ou 5,
**caractérisé en ce**
**qu'**on fait réagir de la 2-nitrophénylhydrazine correspondante avec un acide 2-oxocarboxylique correspondant pour former de la 2-nitrophénylhydrazone et qu'on réduit ensuite la 2-nitrophénylhydrazone obtenue avec de l'hydrogène par catalyse pour obtenir le composé ciblé de formule générale I.

7. Procédé selon la revendication 6, **caractérisé en ce qu'**on fait réagir la 2-nitrophénylhydrazine avec une solution de fermentation qui comprend un acide 2-oxocarboxylique.

8. Procédé selon la revendication 7, **caractérisé en ce que** la solution de fermentation contient l'acide 2-oxocarboxylique en une proportion d'au moins 10 %, de préférence > 20 %.
